# EUROPÄISCHE PATENTSCHRIFT

(11) **EP 3 337 836 B1**
(45) Veröffentlichungstag und Bekanntmachung des Hinweises auf die Patenterteilung: **31.07.2019**
(21) Anmeldenummer: 16753897.4
(22) Anmeldetag: 15.08.2016
(51) Int. Cl.: C08G 18/02, C08G 18/16, B01J 31/02, C07D 295/037, C07C 53/10, C07C 53/128

(54) **VERFAHREN ZUR MODIFIZIERUNG VON ISOCYANATEN UNTER VERWENDUNG VON CYCLISCHEN AMMONIUMSALZEN ALS KATALYSATOR**
METHOD FOR MODIFYING OF ISOCYANATES USING CYCLIC AMMONIUM SALTS AS CATALYST
PROCEDE DE MODIFICATION D'ISOCYANATES A L'AIDE DE SELS D'AMMONIUM CYCLIQUES COMME CATALYSEURS

(30) Priorität: 17.08.2015 EP 15181246
(43) Veröffentlichungstag der Anmeldung: 27.06.2018
(73) Patentinhaber: Covestro Deutschland AG, 51373 Leverkusen (DE)
(72) Erfinder: RICHTER, Frank, 51373 Leverkusen (DE)
(74) Vertreter: Levpat
(86) Internationale Anmeldenummer: PCT/EP2016/069353
(87) Internationale Veröffentlichungsnummer: WO 2017/029266

(56) Entgegenhaltungen:
- EP-A1- 0 962 455
- WO-A1-2011/060407

## Beschreibung

Die Erfindung betrifft ein Verfahren zur Modifizierung von Isocyanaten, bei dem mindestens ein organisches Isocyanat mit einer NCO-Funktionalität > 1 in Gegenwart mindestens eines Katalysators oligomerisiert wird, sowie die Verwendung eines derartigen Katalysators.

Die Oligo- bzw. Polymerisierung von Isocyanaten insbesondere unter Bildung von höhermolekularen Oligomerengemischen mit Uretdion- ("Dimer"), Isocyanurat- ("Trimer") und/oder Iminooxadiazindionstrukturen ("asymmetrisches Trimer") im Molekülgerüst ist seit langem bekannt. Wie vorstehend erkennbar, liegen der Oligo- bzw. Polymerisierung von Isocyanaten prinzipiell die gleichen chemischen Reaktionen zugrunde. Die Umsetzung einer kleineren Anzahl von Isocyanaten miteinander bezeichnet man als Oligomerisierung. Die Umsetzung einer größeren Anzahl von Isocyanaten bezeichnet man als Polymerisierung. Im Rahmen der vorliegenden Erfindung wird die vorstehend beschriebene Oligomerisierung bzw. Polymerisierung von Isocyanaten zusammenfassend als Isocyanatmodifizierung oder Modifizierung von Isocyanaten bezeichnet.

Enthalten die modifizierten Polyisocyanate freie NCO-Gruppen, die ggf. auch mit Blockierungsmitteln vorübergehend desaktiviert worden sein können, sind sie außerordentlich hochwertige Ausgangsstoffe für die Herstellung einer Vielzahl von Polyurethan-Kunststoffen und Beschichtungsmitteln.

Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert) und das erhaltene Polyisocyanat in der Regel vom nicht umgesetzten Monomer abtrennt. Eine Zusammenstellung dieser Verfahren des Standes der Technik findet sich in H. J. Laas et al., J. Prakt. Chem. 1994, 336, 185 ff.

Als Modifizierungskatalysatoren haben sich ionisch aufgebaute Verbindungen bewährt, da sie in sehr geringer Menge, relativ zum umzusetzenden Monomer, eingesetzt werden können und äußerst schnell zum gewünschten Ergebnis führen.

Die Möglichkeit, als Kation zum gegenüber Isocyanaten katalytisch aktiven Anion wie Hydroxid, Alkanoat, Alkoxylat etc. auch Tetraorganylammonium oder -phosphonium zu verwenden, ist allgemein bekannt, wenngleich in der Regel nicht explizit als besonders bevorzugt herausgehoben, vgl.: H. J. Laas et al., J. Prakt. Chem. 1994, 336, 185 ff.

Weiterhin ist die Verwendung von Fluoriden und Hydrogenpolyfluoriden, letztere sind stabile Addukte von HF an Fluorid-Ionen enthaltenden Verbindungen, optional auch in Form ihrer Ammonium- oder Phosphoniumsalze, für die Isocyanatmodifizierung u.a. bekannt aus den EP 962 455 A1, EP 962 454 A1, EP 896 009 A1, EP 798 299 A1, EP 447 074 A1, EP 379 914 A1, EP 339 396 A1, EP 315 692 A1, EP 295 926 A1 sowie EP 235 388 A1.

Allerdings weisen die Tetraorganylammonium und -phosphonium (Hydrogenpoly)fluoride des Standes der Technik bei der Durchführung der Modifizierungsreaktion den Nachteil auf, dass bei ihrer Verwendung die Reaktion mitunter nur durch kontinuierliche Katatalysatordosierung aufrecht zu erhalten ist, d.h. dass die Zersetzung des Katalysators im Isocyanatmilieu im Vergleich zur Modifizierungsreaktion technisch unakzeptabel schnell abläuft.

Hinzu kommt, dass beim Einsatz von Tetraorganylammonium(hydrogen)polyfluoriden mitunter ein untypischer Reaktionsverlauf zu beobachten ist, der zu Produkten mit deutlich niedrigerem Iminooxadiazindiongruppenanteil führt, als bei regulärem Verlauf der Wärmeproduktionsrate (vgl. EP 962 455 A1). Dieser Nachteil wurde nach der Lehre der EP 962 455 A1 durch den Einsatz von Phosphoniumsalzen eliminiert, allerdings weisen letztere - insbesondere bei höheren Reaktionstemperaturen - die weiter oben erwähnte, unakzeptabel hohe Zersetzungstendenz auf, wobei die Zersetzungsprodukte einen nachteiligen Einfluß auf die Prozeß- und Produktstabilität haben können.

EP 2 415 795 A1 beansprucht u.a. sehr stabile Tetraorganylphosphonium(hydrogenpoly)fluoride, die diese Nachteile nicht aufweisen, allerdings kommerziell nicht verfügbar und nur aufwendig herzustellen sind.

Der Erfindung lag daher die Aufgabe zugrunde, ein verbessertes Verfahren zur Isocyanatmodifizierung zur Verfügung zu stellen, bei dem kommerziell gut zugängliche oder aus preiswerten Edukten leicht herstellbare Verbindungen als Katalysatoren eingesetzt werden, die eine hohe katalytische Aktivität und Selektivität bei gleichzeitig guter Katalysatorstabilität aufweisen.

Diese Aufgabe wurde erfindungsgemäß gelöst durch ein Verfahren zur Modifizierung von Isocyanaten, bei dem mindestens ein organisches Isocyanat mit einer NCO-Funktionalität > 1 in Gegenwart mindestens eines Katalysators oligomerisiert wird, dadurch gekennzeichnet, dass der Katalysator mindestens ein cyclisches Ammoniumsalz mit einem Kation der Formel I als Katalysatoren für die Isocyanatmodifizierung umfasst, 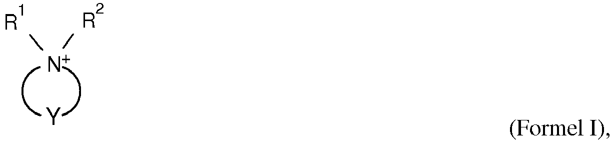 wobei die N-ständigen Substituenten R¹ und R² unabhängig voneinander für gleiche oder verschiedene aliphatische, cycloaliphatische, aromatische oder araliphatische C₁-C₂₀-Reste stehen, die gesättigt oder ungesättigt, linear oder verzweigt, ggf. substituiert und/oder durch Heteroatome aus der Reihe Sauerstoff, Schwefel, Stickstoff unterbrochen sind und Y für ein substituiertes oder unsubstituiertes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht.

Bevorzugt bedeuten die Bezugnahmen auf "umfassend", "enthaltend" usw. "im Wesentlichen bestehend aus" und ganz besonders bevorzugt "bestehend aus".

Verbindungen der Formel I sind in einfacher Weise nach literaturbekannten Methoden (z.B. Houben-Weyl, Methoden der Organischen Chemie, 4. Aufl., Bd. XI/2, S. 591-640 und darin zit. Literatur) beispielsweise durch Alkylierung tertiärer cyclischer Amine R¹NY bzw. R²NY, wobei Y für vorstehend genanntes cyclisches Segment steht, mit Halogenalkanen R²-Hal, bzw. R¹-Hal und anschließendem Anionenaustausch zugänglich, wobei Halogen für Cl, Br und I, bevorzugt Cl, steht. Alternativ kann beispielsweise bei Carboxylaten sowie Carbonaten auch unmittelbar mit den Carbonsäureestern alkyliert werden, die R¹ bzw. R² im Esterrest tragen, hierbei sind Methylester bevorzugt.

Alternativ kann das N-haltige Ringsegment auch im Zuge einer (ggf. simultan geführten) Alkylierungs/Quaternisierungsreaktionsfolge an Ammoniak sowie primären bzw. sekundären Aminen der Formeln R¹NH₂, R²NH₂ bzw. R¹,R²NH gewonnen werden.

In einer ersten bevorzugten Ausführungsform stehen R¹ und R² in Formel I unabhängig voneinander für gleiche oder verschiedene C₁-C₈-Alkylsubstituenten, bevorzugt für gleiche oder verschiedene C₁-C₆-Alkylsubstituenten und sind besonders bevorzugt linear aufgebaut. Diese sind z.B. in einfacher Weise durch Reaktion von ggf. C-substituierten Pyrrolidinen, Piperidinen und Azepanen (1H-Hexahydroazepinen) mit 1-Halogenalkanen, wie z.B. Chlormethan, Brommethan, Iodmethan, Chlorethan, Bromethan, Iodethan, 1-Halogenpropanen, 1-Halogenbutanen etc. sowie derer C-substituierter Derivate zugänglich, wobei Halogen für Cl, Br und I, bevorzugt Cl, steht.

In einer weiteren bevorzugten Ausführungsform stehen R¹ und R² in Formel I unabhängig voneinander für gleiche oder verschiedene, ggf. am aromatischen Kern substituierte, Benzyl-Reste.

Weiterhin sind beispielsweise durch analoge Umsetzung von an N durch R¹ oder R² substituierten Verbindungen (ggf. C-substituierten Oxazolidinen, Isoxazolidinen, Oxazinanen, Morpholinen und Oxazepanen sowie den Analoga der vorstehend genannten N-O-Heterocyclen, die S statt O enthalten, weiterhin Imidazolidinen, Pyrazolidinen, Piperazinen und strukturell verwandten Verbindungen) mit vorstehend genannten Halogenalkanen auch Verbindungen zugänglich, die im Segment Y der Formel I ein durch Heteroatome unterbrochenes Segment aufweisen.

Das Segment Y in Formel I kann selbstverständlich noch weitere Ringe aufweisen. Beispielhaft seien genannt: am N-Atom mit R¹ bzw. R² subsituierte 8-Azabicyclo[3.2.1]octane sowie 2-Azatricyclo[3.3.1.1^{3,7}]decane (2-Alkyl-2-aza-adamantane) und Derivate dieser.

Bei den zwei oder mehr Stickstoffatome enthaltenden Spezies besteht darüber hinaus die Möglichkeit, durch entsprechende Variation der Reaktionsbedingungen auch Salze mit zwei- bzw. mehrfach geladenem Kation zu erzeugen oder durch vorherige geeignete Substitution des bzw. der N-Atome zu einfach positiv geladenen Kationen der Formel I zu gelangen in denen sich ein bzw. mehrere exocyclische(r) Alkylsubstituent(en) an dem bzw. den dreibindigen N-Atom(en) des Ringes Y befindet bzw. befinden.

Natürlich lässt sich auch durch geeignete Wahl des Alkylierungsmittels eine strukturelle Variation in das Ringsegment Y einbringen, beispielhaft seien Reaktionen von Bis(2-halogenethyl)ethern mit den o.g. sekundären Aminen R¹,R²NH genannt.

In einer weiteren bevorzugten Ausführungsform steht das Segment Y für ggf. substituierte und/oder durch Heteroatome aus der Reihe Sauerstoff, Schwefel, Stickstoff sowie aromatische Ringe unterbrochene C₄-C₆-Alkylenketten und ist insbesondere linear aufgebaut.

Als Anionen können bei den Verbindungen der Formel I prinzipiell alle Spezies zum Einsatz kommen, die als katalytisch aktiv gegenüber Isocyanaten bekannt sind, bevorzugt sind das Hydroxid, Alkanoat, Carboxylat, Heterocyclen mit mindestens einem negativ geladenen Stickstoffatom im Ring, insbesondere Azolat, Imidazolat, Triazolat, Tetrazolat, Fluorid, Hydrogendifluorid, höhere Polyfluoride oder Mischungen von diesen, wobei die Fluoride, Hydrogendifluoride und höheren Polyfluoride (Addukte von mehr als einem Äquivalent HF an Fluorid-Ionen enthaltenden Verbindungen) erfindungsgemäß zu Produkten mit hohem Iminooxadiazindiongruppengehalt führen.

Die erfindungsgemäßen Katalysatoren können einzelnen oder in beliebigen Mischungen untereinander verwendet werden. So liegen die Lösungen quaternärer Ammoniumhydroxide in verschiedenen Alkoholen je nach pKs-Wert der Base und des verwendeten Alkohols teilweise oder vollständig als Ammoniumsalze mit Alkoholat-Anion vor. Dieses Gleichgewicht kann durch Entfernung des aus dieser Reaktion resultierenden Reaktionswassers ganz auf die Seite vollständiger Alkoholatbildung verschoben werden. Als Methoden zur Wasserentfernung eignen sich dabei alle aus der Literatur hierfür bekannten Methoden, insbes. die Azeotropdestillation ggf. unter Zuhilfenahme eines geeigneten Schleppmittels.

Mit dem erfindungsgemäßen Modifizierverfahren ist ganz allgemein eine breite Palette qualitativ hochwertiger und deshalb für den Polyurethansektor sehr wertvoller Polyisocyanate in einfacher Weise zugänglich. Abhängig vom verwendeten Ausgangs(di)isocyanat und den Reaktionsbedingungen entstehen beim erfindungsgemäßen Verfahren Polyisocyanate vom sog. Isocyanat-Trimertyp (d.h. enthaltend Isocyanurat- und/oder Iminooxadiazindionstrukturen) mit geringem Anteil an Uretdiongruppen ("Isocyanat-Dimere"). Bei steigender Reaktionstemperatur steigt der Anteil letzterer in den Verfahrensprodukten in der Regel an, allerdings ist dieser Effekt weit weniger ausgeprägt, als bei Verwendung von Phosphoniumsalzen mit identischem Anion.

Bei dem erfindungsgemäßen Verfahren kann weiter vorgesehen sein, dass die Oligomerisierung unter Anwesenheit eines Lösungsmittels und/oder Additivs durchgeführt wird.

Zur Durchführung des erfindungsgemäßen Verfahrens können prinzipiell alle bekannten Mono-, Di- oder Polyisocyanate des Standes der Technik einzeln oder in beliebigen Mischungen untereinander eingesetzt werden.

Beispielhaft seien genannt: Pentamethylendiisocyanat (PDI), Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H₆XDI), 2,4- sowie 2,6-Toluylendiisocyanat (TDI), Bis(4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly)amine in die entsprechenden (Poly)isocyanate zugänglich sind (Polymer-MDI).

Bevorzugt sind aliphatische Diisocyanate, d.h. Diisocyanate bei denen beide NCO-Gruppen an ein sp3-hybridisiertes Kohlenstoffatom gebunden sind.

Besonders bevorzugt sind Pentamethylendiisocyanat (PDI), Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H₆XDI).

Es ist belanglos, nach welchen Verfahren die vorstehend genannten Isocyanate generiert werden, d.h. mit oder ohne Verwendung von Phosgen.

Die Menge des im erfindungsgemäßen Verfahren einzusetzenden Katalysators richtet sich in erster Linie nach dem verwendeten organischen Isocyanat und der angestrebten Reaktionsgeschwindigkeit und liegt vorzugsweise zwischen ≥ 0,001 und ≤ 5 mol-%, bevorzugt zwischen ≥ 0,002 und ≤ 2 mol-%, bezogen auf die Summe der Stoffmengen des eingesetzten Isocyanates und des Katalysators.

Der Katalysator kann im erfindungsgemäßen Verfahren unverdünnt oder in Lösungsmitteln gelöst eingesetzt werden. Als Lösungsmittel kommen dabei alle Verbindungen in Frage, die nicht mit dem Katalysator reagieren und ihn in ausreichendem Maße zu lösen vermögen, z.B. ggf. halogenierte, aliphatische oder aromatische Kohlenwasserstoffe, Alkohole, Ketone, Ester sowie Ether. Bevorzugt werden Alkohole verwendet.

Das erfindungsgemäße Verfahren kann im Temperaturbereich von 0 °C bis + 250 °C, bevorzugt 20 °C bis 200 °C, besonders bevorzugt 40 °C bis 150 °C erfolgen und bei beliebigen Umsetzungsgraden, bevorzugt nachdem 5 bis 80 %, besonders bevorzugt 10 bis 60 %, des eingesetzten Isocyanates umgesetzt wurden, unterbrochen werden.

Zur Katalysatordeaktivierung bieten sich prinzipiell eine ganze Reihe vorbeschriebener Methoden des Standes der Technik an, wie z.B. die Zugabe (unter- oder über-) stöchiometrischer Mengen an Säuren oder Säurederivaten (z.B. Benzoylchlorid, saure Ester Phosphor oder Schwefel enthaltender Säuren, diese Säuren selbst etc., nicht jedoch HF), adsorptive Bindung des Katalysators und anschließende Abtrennung durch Filtration und andere dem Fachmann bekannte Methoden.

In einer weiteren bevorzugten Ausführungsform wird nicht umgesetztes organisches Isocyanat nach Desaktivierung des Katalysatorsystems nach einem beliebigen Verfahren des Standes der Technik, z.B. durch (Dünnschicht)destillation oder Extraktion, abgetrennt und bevorzugt anschließend wiederverwendet.

Im Gegensatz zur Katalyse durch Ammoniumsalze in denen das ladungstragende Stickstoffatom nicht Teil eines Ringsystems ist, beobachtet man bei Einsatz der erfindungsgemäßen Katalysatoren mit Fluorid- bzw. Oligo/Polyfluoridanionen überraschenderweise keinerlei Anomalien in der Wärmeproduktionsrate und immer einen gleichmäßigen Reaktionsverlauf der zu qualitativ hochwertigen Produkten mit für die jeweiligen Reaktionsbedingungen optimalem Iminooxadiazindiongruppengehalt führt.

Ganz allgemein sind die erfindungsgemäßen Katalysatoren unabhängig vom Anion, das für die katalytische Aktivität und Selektivität verantwortlich ist, im umzusetzenden organischen Isocyanat wesentlich stabiler als die literaturbekannten Derivate des Standes der Technik.

Nach einer besonderen, kontinuierlich arbeitenden Ausführungsform des erfindungsgemäßen Verfahrens kann die Oligomerisierung kontinuierlich, z.B. in einem Rohrreaktor vorgenommen werden.

Die nach dem erfindungsgemäßen Verfahren erhältlichen Produkte bzw. Produktgemische stellen mithin vielseitig verwendbare Ausgangsmaterialien zur Herstellung von, ggf. geschäumte(n), Kunststoffe(n) sowie Lacken, Beschichtungsmitteln, Klebstoffen und Zuschlagstoffen dar. Insbesondere zur Herstellung von, ggf. wasserdipergierbaren Ein- und Zweikomponenten-Polyurethanlacken eignen sie sich, ggf. in NCO-blockierter Form, aufgrund ihrer im Vergleich zu (überwiegend) Isocyanurat-Polyisocyanat basierenden Produkten verringerten Lösungs- sowie Schmelzviskosität bei ansonsten gleich hohem bzw. verbessertem Eigenschaftsprofil. So sind die erfindungsgemäßen Verfahrensprodukte auf HDI-Basis auch in hoher Verdünnung in Lacklösungsmitteln stabiler gegen das Auftreten von Ausflockungen bzw. Trübungen, als entsprechende Produkte des Standes der Technik.

Die erfindungsgemäßen Verfahrensprodukte können als solche oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen ggf. mit Blockierungsmitteln desaktiviert wurden, eingesetzt werden.

Ein weiterer Gegenstand der vorliegenden Erfindung ist gerichtet auf die Verwendung eines cylischen Ammoniumsalzes mit einem Kation der Formel I, 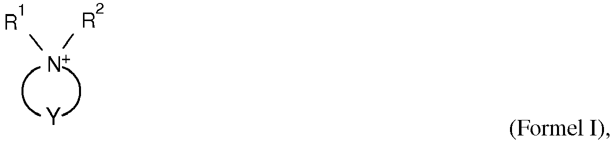 wobei die N-ständigen Substituenten R¹ und R² unabhängig voneinander für gleiche oder verschiedene aliphatische, cycloaliphatische, aromatische oder araliphatische C₁-C₂₀-Reste stehen, die gesättigt oder ungesättigt, linear oder verzweigt, ggf. substituiert und/oder durch Heteroatome aus der Reihe Sauerstoff, Schwefel, Stickstoff unterbrochen sind und Y für ein substituiertes oder unsubstituiertes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht, als Katalysatoren für die Oligomerisierung von organischen Isocyanaten mit einer NCO-Funktionalität > 1.

Die nachfolgenden Vergleichsbeispiele und Beispiele sollen die Erfindung näher erläutern, ohne sie jedoch einzuschränken.

### Beispiele:

Alle Prozentangaben sind, soweit nicht anders vermerkt, als Gewichtsprozent zu verstehen.

Mol-% Angaben wurden NMR-spektroskopisch ermittelt und beziehen sich immer, so nicht anders ausgewiesen, auf die Summe der NCO-Folgeprodukte. Die Messungen erfolgten auf den Geräten DPX 400 bzw. DRX 700 der Fa. Brucker an ca. 5 %igen (¹H-NMR) bzw. ca. 50 %igen (¹³C-NMR) Proben in trockenem C₆D₆ bei einer Frequenz von 400 bzw. 700 MHz (¹H-NMR) oder 100 bzw. 176 MHz (¹³C-NMR). Als Referenz für die ppm-Skale wurden geringe Mengen von Tetramethylsilan im Lösungsmittel mit 0 ppm ¹H-NMR-chem. Verschiebung herangezogen. Alternativ wurde auf das Signal des im Lösungsmittel enthaltenen C₆D₅H referenziert: 7,15 ppm ¹H-NMR-chem. Verschiebung, 128,02 ppm ¹³C-NMR-chem. Verschiebung. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit sowie EP 896 009 A1).

Die dynamischen Viskositäten wurden bei 23 °C mit dem Viskosimeter VT 550 der Fa. Haake nach der DIN EN ISO 3219 bestimmt. Durch Messungen bei unterschiedlichen Schergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Ermittlung des NCO-Gehaltes erfolgte durch Titration gemäß DIN EN ISO 11909.

Die Bestimmung der Restmonomergehalte erfolgte gaschromatographisch nach der DIN EN ISO 10283 mit internem Standard.

Alle Reaktionen wurden, wenn nicht anders angegeben, unter einer Stickstoffatmosphäre durchgeführt.

Die verwendeten Diisocyanate sind Produkte der Bayer MaterialScience AG, D-51368 Leverkusen, alle anderen kommerziell zugänglichen Chemikalien wurden von der Fa. Aldrich, D-82018 Taufkirchen, bezogen.

Die nicht kommerziell erhältlichen Katalysatoren wurden nach literaturbekannten Methoden gewonnen (vgl. Houben-Weyl, Methoden der Organischen Chemie, 4. Aufl., Bd. XI/2, S. 591 ff und darin zit. Literatur).

### Beispiele 1 bis 4 Katalysatorherstellung

Zu jeweils 17,8 g (0,1 mol) N-Butyl-N-methylpyrrolidiniumchlorid, gelöst in ca. 65 g 2-Propanol (Beispiel la) bzw. ca. 180 g 2-Ethylhexanol (Beispiele 1b - 1d), wurden tropfenweise bei Zimmertemperatur gegeben:
a) ca. 6,0 g Kaliumhydroxid, als gesättigte Lösung in Methanol,
b) ca. 12 g Kaliumfluorid, als gesättigte Lösung in Methanol
c) ca. 10 g Kaliumacetat, als gesättigte Lösung in Methanol bzw.
d) ca. 80 g einer ca. 20%igen Kaliumpivalatlösung in Methanol

Diese Mischungen wurden ca. 24 h gerührt und anschließend filtriert. Der Filterrückstand wurde dreimal mit ca. 50 ml 2-Propanol gewaschen, wobei nach jedem Waschen im Vakuum bei Zimmertemperatur eingeengt wurde, um Methanol (und in Beispielen 1b - le auch 2-Propanol) weitgehend zu entfernen und Reste anorganischer Salze auszufällen. Anschließend wurde erneut filtriert und mit jeweils ca. 10 ml 2-Propanol gewaschen und aufkonzentriert wie vorher. Die jeweiligen vereinigten Filtrate wurden schließlich im Vakuum auf ca. 80 g (Beispiel 1a) bzw. ca. 150 g (Beispiele 1b - d) eingeengt und analysiert.
a) OH⁻- Gehalt* (titrimetrisch gg. 0,1 N HCl, Indikator Phenolphthalein): 2,0 %*
b) F⁻ - Gehalt (ionensensitive Elektrode bei pH-Wert = 5,5): 1,1 %.
c) CH₃C(O)O⁻ - Gehalt (titrim. gg. 0,1 N HCl, Indikator Bromphenolblau): 3,8 %
d) (CH₃)₃CC(O)O⁻ - Gehalt (titrim. gg. 0,1 NHCl, Indikator Bromphenolblau): 6,4 %.

Katalysatorlösung 1b wurde anschließend mit 1,8 g wasserfreier HF zur Überführung in das Difluorid versetzt (Katalysatorlösung le). * NMR-spektroskopisch finden sich bei den Lösungen der Hydroxide gelegentlich Hinweise auf das (anteilige) Vorliegen des dem Lösemittel entsprechenden Alkoholates (2-Propanolat bzw. 2-Ethylhexanoat). Hier wird der Einfachheit halber immer auf das Hydroxid-Ion abgestellt, es kann jedoch auch - anteilig oder ausschließlich - das Alkanoat-Anion in Lösung vorliegen.

Weitere Katalysatoren 2 - 4 wurden nach analogem Verfahren aus dem entsprechenden quaternären Ammoniumchlorid gewonnen, welches seinerseits ggf. vorher aus dem jeweiligen tertiären cyclischen Amin und dem entsprechenden Chloralkan dargestellt worden war. Höhere Oligo-fluoride wurden durch Zugabe entsprechender HF-Überschüsse zu den in Analogie zu 1b) erhaltenen Fluoridlösungen erhalten.

Anschließend wurde die optimale Katalysatorkonzentration für die Isocyanat-Trimerisierung in orientierenden Vorversuchen bei 60 °C mit HDI (vgl. Beispiel 5) ermittelt und die Konzentration der Katalysatorlösung durch Verdünnen mit 2-Propanol bzw. 2-Ethylhexanol (im weiteren Text: 2-EH) so eingestellt, dass keine bzw. nur geringe Gelteilchenbildung bei der Zugabe der Katalysatorlösung zum HDI zu beobachten war. Eine Übersicht hierzu findet sich in Tabelle 1. Dort wird in der Spalte "Lösemittel" nur der ggf. zur weiteren Verdünnung der Ausgangslösung zugesetzte Alkohol aufgeführt. Wurden die nach der für die Katalysatoren 1a bis le oben beschriebenen Verfahrensweise erhaltenen Katalysatoren ohne weitere Verdünnung eingesetzt, ist das Lösemittel fast ausschließlich 2-EH mit geringen Resten an 2-Propanol und ggf. Methanol (Ausnahme: Beispiel 1a: ausschließlich 2-Propanol und ggf. Methanol).

**Tabelle 1: Übersicht der hergestellten Katalysatoren (die Katalysatorkonzentration bezieht sich auf den Wirkstoff (Kation und Anion))**

| Beispiel | Kation | Anion | Lösemittel | Konzentration [%] |
|---|---|---|---|---|
| 1a | 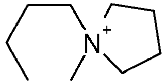 | OH⁻ | 2-PrOH | 20 |
| 1c | 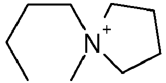 | CH₃C(O)O⁻ | 2-EH | 2 |
| 1d | 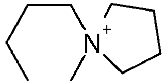 | (CH₃)₃CC(O)O⁻ | 2-EH | 4 |
| 1e | 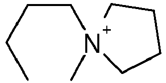 | [HF₂]⁻ | 2-EH | 8 |
| 2 | 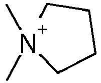 | OH⁻ | 2-PrOH | 20 |
| 3 | 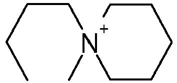 | [HF₂]⁻ | 2-EH | 8 |
| 4 | 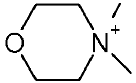 | [F^{∗}1,35 HF]⁻ | 2-PrOH | 15 |

### Beispiele 5 bis 11 - erfindungsgemäße Isocyanat-Modifizierungen

In einem doppelwandigen, durch einen externen Kreislauf auf die jeweils gewünschte Starttemperatur temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (< 1 mbar) von gelösten Gasen befreit. Nach Belüften mit Stickstoff wurde die in Tabelle 2 angegebene Katalysatorart und Katalysatormenge ggf. portionsweise so dosiert, dass die in Tabelle 2 angegebene Maximaltemperatur nicht überschritten wurde. Nachdem ca. 1 mol Isocyanatgruppen umgesetzt waren, indiziert durch Erreichen eines NCO-Gehaltes um 45,8 %, wurde der Katalysator durch Zugabe einer zum Katalysator äquivalenten Menge des in Tabelle 2 angegebenen Stoppers deaktiviert, weitere 30 min bei Reaktionstemperatur nachgerührt und anschließend aufgearbeitet. Die Zeit zwischen (erster) Katalysatorzugabe und Stopperzugabe ist in Tabelle 2 als Reaktionszeit vermerkt.

Die Aufarbeitung erfolgte durch Vakuumdestillation in einem Dünnschichtverdampfer, Typ Kurzwegverdampfer (KWV), mit vorgeschaltetem Vorverdampfer (VV) (Destillationsdaten: Druck: 0,08 +/- 0,04 mbar, VV-Temperatur: 120 °C, HV-Temp.: 140 °C), wobei nicht umgesetztes Monomer als Destillat und das monomerenarme Polyisocyanatharz als Sumpfprodukt separiert wurden (Startdurchlauf). Das Polyisocyanatharz wurde separiert und das Destillat in einer zweiten Planschliff-Rührapparatur, die identisch zur ersten aufgebaut ist, gesammelt und mit frisch entgastem HDI auf die Ausgangsmenge (1000 g) aufgefüllt. Anschließend wurde erneut katalysiert und verfahren wie eingangs beschrieben. Diese Verfahrensweise wurde, ggf. unter Variation der Reaktionstemperatur, mehrmals wiederholt (Versuche A, B, C, usw.). Die Ergebnisse sind Tabelle 2 zu entnehmen.

Abschließend wurde die Destillatzusammensetzung gaschromatografisch ermittelt. In keinem Fall konnten Zersetzungsprodukte des Katalysatorkations detektiert werden (Nachweisgrenze ca. 20 ppm).

**Tabelle 2: Durchgeführte Isocyanatmodifizierungen**

| Beispiel | | Katalysator (s. Tabelle 1) | Katalysatormenge [g] | Reaktionstemperatur von - bis [°C] | | Stopper* | Reaktionszeit [min] |
|---|---|---|---|---|---|---|---|
| 5 | A | 1a | 1,45 | 60 | 65 | 3 | 60 |
| 5 | B | 1a | 1,16 | 60 | 83 | 3 | 4 |
| 5 | C | 1a | 1,03 | 80 | 92 | 3 | 17 |
| 5 | D | 1a | 0,89 | 80 | 85 | 3 | 20 |
| 5 | E | 1a | 0,65 | 100 | 200 | 3 | 2¹⁾ |
| 5 | F | 1a | 1,14 ¹⁾ | 100 | 125 | 3 | 24 |
| 6 | A | 1c | 15,06 | 60 | 64 | 1 | 60 |
| 6 | B | 1c | 12,15 | 60 | 63 | 1 | 90 |
| 6 | C | 1c | 19,40 | 80 | 88 | 1 | 20 |
| 6 | D | 1c | 16,67 | 80 | 83 | 1 | 30 |
| 6 | E | 1c | 21,92 | 100 | 125 | 1 | 10 |
| 6 | F | 1c | 21,56 | 100 | 104 | 1 | 15 |
| 7 | A | 1d | 4,38 | 60 | 60 | 1 | 230 |
| 7 | B | 1d | 4,26 | 60 | 64 | 1 | 120 |
| 7 | C | 1d | 5,80 | 80 | 85 | 1 | 27 |
| 7 | D | 1d | 5,95 | 80 | 84 | 1 | 25 |
| 7 | E | 1d | 9,94 | 100 | 137 | 1 | 40 |
| 7 | F | 1d | 9,55 | 100 | 101 | 1 | 70 |
| 8 | A | 1e | 1,30 | 60 | 61 | 2 | 42 |
| 8 | B | 1e | 0,94 | 60 | 62 | 2 | 30 |
| 8 | c | 1e | 0,90 | 60 | 61 | 2 | 34 |
| 8 | D | 1e | 1,00 | 60 | 61 | 2 | 28 |
| 8 | E | 1e | 1,08 | 60 | 61 | 2 | 32 |
| 8 | F | 1e | 0,83 | 60 | 61 | 2 | 45 |
| 9 | A | 2 | 0,86 | 60 | 77 | 3 | 14 |
| 9 | B | 2 | 0,90 | 60 | 62 | 3 | 25 |
| 9 | C | 2 | 0,85 | 80 | 88 | 3 | 11 |
| 9 | D | 2 | 0,60 | 80 | 92 | 3 | 7 |
| 9 | E | 2 | 0,59 | 100 | 124 | 3 | 4 |
| 9 | F | 2 | 0,79 | 100 | 103 | 3 | 8 |
| 10 | A | 3 | 3,35 | 60 | 62 | 2 | 40 |
| 10 | B | 3 | 3,46 | 60 | 61 | 2 | 30 |
| 10 | C | 3 | 1,80 | 80 | 85 | 2 | 25 |
| 10 | D | 3 | 1,87 | 80 | 83 | 2 | 15 |
| 10 | E | 3 | 1,03 | 100 | 102 | 2 | 22 |
| 10 | F | 3 | 1,16 | 100 | 105 | 2 | 19 |
| 11 | A | 4 | 0,88 | 60 | 62 | 2 | 42 |
| 11 | B | 4 | 0,84 | 60 | 61 | 2 | 50 |
| 11 | C | 4 | 0,93 | 80 | 83 | 2 | 26 |
| 11 | D | 4 | 0,90 | 80 | 82 | 2 | 18 |
| 11 | E | 4 | 0,92 | 100 | 104 | 2 | 18 |
| 11 | F | 4 | 0,84 | 100 | 104 | 2 | 18 |

| | | | | | | | |
|---|---|---|---|---|---|---|---|
| * Stopper: 1: Dibutylphosphat, 2: Toluolsulfonsäure, 40 %ig in 2-PrOH, 3: Dodecylbenzolsulfonsäure, 70 %ig in 2-PrOH; ¹⁾ aufgrund der sehr hohen Reaktivität und kurzen Reaktionszeit bei extremer Exothermiespitze in Beispiel 5E wurde der Katalysator 1a in Beispiel 5F vorher 1:1 mit 2-Propanol verdünnt. | | | | | | | |

Bei den erhaltenen Harzen handelte es sich ausnahmslos um farbhelle, klare, viskose Flüssigkeiten ohne wahrnehmbaren Amingeruch. Bei Einsatz der fluorhaltigen Katalysatoren resultierten Gemische aus Isocyanurat und Iminooxadiazindion neben wenig Uretdion. Der Anteil an Iminooxadiazindiongruppen hat bei einer Reaktionstemperatur um 60 °C sein Maximum und fällt bei Erhöhung der Reaktionstemperatur ab. Es bilden sich dann verstärkt Isocyanurat und Uretdion, der Anteil Letzterer nimmt allerdings deutlich weniger stark als bei der Katalyse mit den entsprechenden quaternären Phosphoniumsalzen gemäß EP 962 455 A1 zu. Die Sauerstoffenthaltenden Anionen liefern Produkte vom Isocyanurattyp wobei der als Katalysatorlösemittel eingesetzte Alkohol vollständig zum Allophanat umgesetzt wird.

## Patentansprüche

1. Verfahren zur Modifizierung von Isocyanaten, bei dem mindestens ein organisches Isocyanat mit einer NCO-Funktionalität > 1 in Gegenwart mindestens eines Katalysators oligomerisiert wird, **dadurch gekennzeichnet, dass** der Katalysator mindestens ein cyclisches Ammoniumsalz mit einem Kation der Formel I als Katalysatoren für die Isocyanatmodifizierung umfasst, 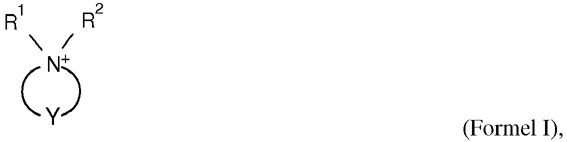 wobei die N-ständigen Substituenten R¹ und R² unabhängig voneinander für gleiche oder verschiedene aliphatische, cycloaliphatische, aromatische oder araliphatische C₁-C₂₀-Reste stehen, die gesättigt oder ungesättigt, linear oder verzweigt, ggf. substituiert und/oder durch Heteroatome aus der Reihe Sauerstoff, Schwefel, Stickstoff unterbrochen sind und Y für ein substituiertes oder unsubstituiertes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht.

2. Verfahren nach Anspruch 1, **dadurch gekennzeichnet, dass** R¹ und R² unabhängig voneinander für gleiche oder verschiedene C₁-C₈-Alkylsubstituenten, bevorzugt für gleiche oder verschiedene C₁-C₆-Alkylsubstituenten stehen und besonders bevorzugt linear aufgebaut sind.

3. Verfahren nach Anspruch 1, **dadurch gekennzeichnet, dass** R¹ und R² in Formel I unabhängig voneinander für gleiche oder verschiedene, ggf. am aromatischen Kern substituierte, Benzyl-Reste stehen.

4. Verfahren nach einem der vorstehenden Ansprüche, **dadurch gekennzeichnet, dass** das Segment Y für ggf. substituierte und/oder durch Heteroatome aus der Reihe Sauerstoff, Schwefel, Stickstoff sowie aromatische Ringe unterbrochene C₄-C₆-Alkylenketten steht und insbesondere linear aufgebaut ist.

5. Verfahren nach einem der vorstehenden Ansprüche, **dadurch gekennzeichnet, dass** das Anion des cylischen Ammoniumsalzes ausgewählt ist aus Hydroxid, Alkanoat, Carboxylat, Heterocyclen mit mindestens einem negativ geladenen Stickstoffatom im Ring, insbesondere Azolat, Imidazolat, Triazolat, Tetrazolat, Fluorid, Hydrogendifluorid, höhere Polyfluoride oder Mischungen von diesen.

6. Verfahren nach einem der vorstehenden Ansprüche, **dadurch gekennzeichnet, dass** die Oligomerisierung unter Anwesenheit eines Lösungsmittels und/oder Additivs durchgeführt wird.

7. Verfahren nach einem der vorstehenden Ansprüche, **dadurch gekennzeichnet, dass** das organische Isocyanat ausgewählt ist aus aliphatischen Diisocyanaten, insbesondere aus Pentamethylendiisocyanat, Hexamethylendiisocyanat, 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3(4)-Isocyanatomethyl-1-methylcyclohexylisocyanat, Isophorondiisocyanat, 1,3- sowie 1,4-Bis(isocyanatomethyl)benzen, 1,3- sowie 1,4-Bis(isocyanatomethyl)cyclohexan oder Mischungen von diesen.

8. Verfahren nach einem der vorstehenden Ansprüche, **dadurch gekennzeichnet, dass** der Katalysator der Formel I in einer Menge zwischen ≥ 0,001 und ≤ 5 mol-%, bevorzugt zwischen ≥ 0,002 und ≤ 2 mol-%, bezogen auf die Summe der Stoffmengen des eingesetzten Isocyanates und des Katalysators, eingesetzt wird.

9. Verfahren nach einem der vorstehenden Ansprüche, **dadurch gekennzeichnet, dass** das Verfahren im Temperaturbereich von 0 °C bis + 250 °C durchgeführt wird, bevorzugt 20 bis 200 °C, besonders bevorzugt 40 bis 150 °C.

10. Verfahren nach einem der vorstehenden Ansprüche, **dadurch gekennzeichnet, dass** die Oligomerisierung abgebrochen wird, nachdem 5 bis 80 Gew.-% des eingesetzten organischen Isocyanats umgesetzt sind, bevorzugt 10 bis 60 Gew.-%.

11. Verfahren nach Anspruch 10, **dadurch gekennzeichnet, dass** die Oligomerisierung abgebrochen wird, indem der Katalysator desaktiviert wird, insbesondere durch Zugabe einer Säure oder eines Säurederivates wie Benzoylchlorid, eines sauren Esters Phosphor oder Schwefel enthaltender Säuren, diese Säuren selbst, adsorptive Bindung des Katalysators und anschließende Abtrennung durch Filtration oder Kombinationen hiervon.

12. Verfahren nach Anspruch 10 oder 11, **dadurch gekennzeichnet, dass** nicht umgesetztes organisches Isocyanat aus der Reaktionsmischung abgetrennt wird.

13. Verwendung eines cyclischen Ammoniumsalzes mit einem Kation der Formel I, 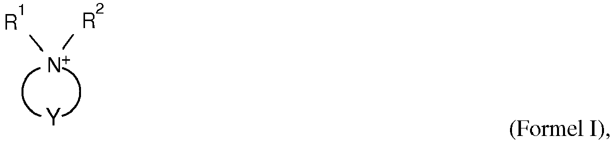 wobei die N-ständigen Substituenten R¹ und R² unabhängig voneinander für gleiche oder verschiedene aliphatische, cycloaliphatische, aromatische oder araliphatische C₁-C₂₀-Reste stehen, die gesättigt oder ungesättigt, linear oder verzweigt, ggf. substituiert und/oder durch Heteroatome aus der Reihe Sauerstoff, Schwefel, Stickstoff unterbrochen sind und Y für ein substituiertes oder unsubstituiertes, lineares oder verzweigtes, ggf. durch Heteroatome aus der Reihe Sauerstoff, Schwefel, Stickstoff sowie aromatische Ringe unterbrochenes, ggf. weitere Ringe aufweisendes, C₂-C₂₀-Segment steht, als Katalysatoren für die Oligomerisierung von organischen Isocyanaten mit einer NCO-Funktionalität > 1.

## Claims

1. Process for modifying isocyanates, in which at least one organic isocyanate having an NCO functionality of > 1 is oligomerized in the presence of at least one catalyst, **characterized in that** the catalyst comprises at least one cyclic ammonium salt having a cation of the formula I as catalysts for the isocyanate modification, 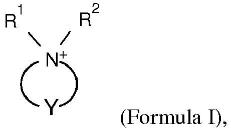 wherein the N-substituents R¹ and R² are mutually independently identical or different aliphatic, cycloaliphatic, aromatic or araliphatic C₁-C₂₀ radicals, which are saturated or unsaturated, linear or branched, optionally substituted and/or interrupted by heteroatoms from the group of oxygen, sulfur and nitrogen and Y is a substituted or unsubstituted, linear or branched C₂-C₂₀ segment optionally interrupted by heteroatoms from the group of oxygen, sulfur, nitrogen and also by aromatic rings, and optionally comprising further rings.

2. Process according to Claim 1, **characterized in that** R¹ and R² are mutually independently identical or different Ci-Cs-alkyl substituents, preferably identical or different C₁-C₆-alkyl substituents and particularly preferably have a linear structure.

3. Process according to Claim 1, **characterized in that** R¹ and R² in formula I are mutually independently identical or different benzyl radicals optionally substituted on the aromatic ring.

4. Process according to any of the preceding claims, **characterized in that** segment Y is C₄-C₆-alkylene chains optionally substituted and/or interrupted by heteroatoms from the group of oxygen, sulfur, nitrogen and also by aromatic rings and especially has a linear structure.

5. Process according to any of the preceding claims, **characterized in that** the anion of the cyclic ammonium salt is selected from hydroxide, alkanoate, carboxylate, heterocycles having at least one negatively charged nitrogen atom in the ring, especially azolate, imidazolate, triazolate, tetrazolate, fluoride, hydrogendifluoride, higher polyfluorides or mixtures of these.

6. Process according to any of the preceding claims, **characterized in that** the oligomerization is conducted in the presence of a solvent and/or additive.

7. Process according to any of the preceding claims, **characterized in that** the organic isocyanate is selected from aliphatic diisocyanates, especially from pentamethylene diisocyanate, hexamethylene diisocyanate, 2-methylpentane 1,5-diisocyanate, 2,4,4-trimethylhexane 1,6-diisocyanate, 2,2,4-trimethylhexane 1,6-diisocyanate, 4-isocyanatomethyloctane 1,8-diisocyanate, 3(4)-isocyanatomethyl-1-methylcyclohexyl isocyanate, isophorone diisocyanate, 1,3- and 1,4-bis(isocyanatomethyl)benzene, 1,3- and 1,4-bis(isocyanatomethyl)cyclohexane or mixtures of these.

8. Process according to any of the preceding claims, **characterized in that** the catalyst of the formula I is used in an amount between ≥ 0.001 and ≤ 5 mol%, preferably between ≥ 0.002 and ≤ 2 mol%, based on the sum total of the molar amounts of the isocyanate used and of the catalyst.

9. Process according to any of the preceding claims, **characterized in that** the process is conducted within the temperature range from 0°C to +250°C, preferably 20 to 200°C, more preferably 40 to 150°C.

10. Process according to any of the preceding claims, **characterized in that** the oligomerization is stopped after 5% to 80% by weight of the organic isocyanate used has been converted, preferably 10% to 60% by weight.

11. Process according to Claim 10, **characterized in that** the oligomerization is stopped by deactivating the catalyst, especially by addition of an acid or an acid derivative such as benzoyl chloride, an acidic ester of phosphorus- or sulfur-containing acids, these acids themselves, adsorptive binding of the catalyst and subsequent removal by filtration or combinations thereof.

12. Process according to Claim 10 or 11, **characterized in that** unconverted organic isocyanate is removed from the reaction mixture.

13. Use of a cyclic ammonium salt having a cation of the formula I, 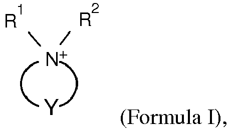 wherein the N-substituents R¹ and R² are mutually independently identical or different aliphatic, cycloaliphatic, aromatic or araliphatic C₁-C₂₀ radicals, which are saturated or unsaturated, linear or branched, optionally substituted and/or interrupted by heteroatoms from the group of oxygen, sulfur and nitrogen and Y is a substituted or unsubstituted, linear or branched C₂-C₂₀ segment optionally interrupted by heteroatoms from the group of oxygen, sulfur, nitrogen and also by aromatic rings, and optionally comprising further rings, as catalysts for the oligomerization of organic isocyanates having an NCO functionality of > 1.

## Revendications

1. Procédé de modification d'isocyanates, selon lequel au moins un isocyanate organique ayant une fonctionnalité NCO > 1 est oligomérisé en présence d'au moins un catalyseur, **caractérisé en ce que** le catalyseur comprend au moins un sel d'ammonium cyclique avec un cation de la formule I en tant que catalyseurs pour la modification d'isocyanates 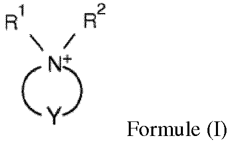 dans laquelle les substituants R¹ et R² sur le N représentent indépendamment l'un de l'autre des radicaux en C₁-C₂₀ aliphatiques, cycloaliphatiques, aromatiques ou araliphatiques identiques ou différents, qui sont saturés ou insaturés, linéaires ou ramifiés, éventuellement substitués et/ou interrompus par des hétéroatomes de la série constituée par l'oxygène, le soufre, l'azote, et Y représente un segment en C₂-C₂₀ substitué ou non substitué, linéaire ou ramifié, éventuellement interrompu par des hétéroatomes de la série constituée par l'oxygène, le soufre, l'azote et des cycles aromatiques, comprenant éventuellement des cycles supplémentaires.

2. Procédé selon la revendication 1, **caractérisé en ce que** R¹ et R² représentent indépendamment l'un de l'autre des substituants alkyle en C₁-C₈ identiques ou différents, de préférence des substituants alkyle en C₁-C₆ identiques ou différents, et sont de manière particulièrement préférée configurés sous forme linéaire.

3. Procédé selon la revendication 1, **caractérisé en ce que** R¹ et R² dans la formule I représentent indépendamment l'un de l'autre des radicaux benzyle identiques ou différents, éventuellement substitués sur le noyau aromatique.

4. Procédé selon l'une quelconque des revendications précédentes, **caractérisé en ce que** le segment Y représente des chaînes alkylène en C₄-C₆ éventuellement substituées et/ou interrompues par des hétéroatomes de la série constituée par l'oxygène, le soufre, l'azote, ainsi que des cycles aromatiques, et est notamment configuré sous forme linéaire.

5. Procédé selon l'une quelconque des revendications précédentes, **caractérisé en ce que** l'anion du sel d'ammonium cyclique est choisi parmi l'hydroxyde, l'alcanoate, le carboxylate, les hétérocycles contenant au moins un atome d'azote chargé négativement dans le cycle, notamment l'azolate, l'imidazolate, le triazolate, le tétrazolate, le fluorure, l'hydrogénodifluorure, les polyfluorures supérieurs ou les mélanges de ceux-ci.

6. Procédé selon l'une quelconque des revendications précédentes, **caractérisé en ce que** l'oligomérisation est réalisée en présence d'un solvant et/ou d'un additif.

7. Procédé selon l'une quelconque des revendications précédentes, **caractérisé en ce que** l'isocyanate organique est choisi parmi les diisocyanates aliphatiques, notamment parmi le diisocyanate de pentaméthylène, le diisocyanate d'hexaméthylène, le 1,5-diisocyanate de 2-méthylpentane, le diisocyanate de 2,4,4-triméthyl-1,6-hexane, le diisocyanate de 2,2,4-triméthyl-1,6-hexane, le diisocyanate de 4-isocyanatométhyl-1,8-octane, l'isocyanate de 3(4)-isocyanatométhyl-1-méthylcyclohexyle, le diisocyanate d'isophorone, le 1,3- et 1,4-bis(isocyanatométhyl)benzène, le 1,3- et 1,4-bis(isocyanatométhyl)cyclohexane ou les mélanges de ceux-ci.

8. Procédé selon l'une quelconque des revendications précédentes, **caractérisé en ce que** le catalyseur de la formule I est utilisé en une quantité comprise entre ≥ 0,001 et ≤ 5 % en moles, de préférence comprise entre ≥ 0,002 et ≤ 2 % en moles, par rapport à la somme des quantités de matière de l'isocyanate utilisé et du catalyseur.

9. Procédé selon l'une quelconque des revendications précédentes, **caractérisé en ce que** le procédé est réalisé dans la plage de température allant de 0 °C à +250 °C, de préférence de 20 à 200 °C, de manière particulièrement préférée de 40 à 150 °C.

10. Procédé selon l'une quelconque des revendications précédentes, **caractérisé en ce que** l'oligomérisation est interrompue après que 5 à 80 % en poids de l'isocyanate organique utilisé ait été transformé, de préférence 10 à 60 % en poids.

11. Procédé selon la revendication 10, **caractérisé en ce que** l'oligomérisation est interrompue en désactivant le catalyseur, notamment par ajout d'un acide ou d'un dérivé d'acide tel que le chlorure de benzoyle, d'un ester acide d'acides contenant du phosphore ou du soufre, de ces acides eux-mêmes, liaison par adsorption du catalyseur, puis séparation par filtration ou leurs combinaisons.

12. Procédé selon la revendication 10 ou 11, **caractérisé en ce que** l'isocyanate organique non réagi est séparé du mélange réactionnel.

13. Utilisation d'un sel d'ammonium cyclique avec un cation de la formule I : 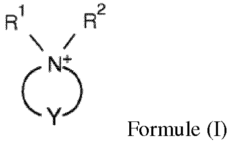 dans laquelle les substituants R¹ et R² sur le N représentent indépendamment l'un de l'autre des radicaux en C₁-C₂₀ aliphatiques, cycloaliphatiques, aromatiques ou araliphatiques identiques ou différents, qui sont saturés ou insaturés, linéaires ou ramifiés, éventuellement substitués et/ou interrompus par des hétéroatomes de la série constituée par l'oxygène, le soufre, l'azote, et Y représente un segment en C₂-C₂₀ substitué ou non substitué, linéaire ou ramifié, éventuellement interrompu par des hétéroatomes de la série constituée par l'oxygène, le soufre, l'azote et des cycles aromatiques, comprenant éventuellement des cycles supplémentaires, en tant que catalyseurs pour l'oligomérisation d'isocyanates organiques ayant une fonctionnalité NCO > 1.
